# EUROPEAN PATENT APPLICATION

(11) **EP 3 918 966 A2**
(43) Date of publication of application: **08.12.2021**
(21) Application number: 21176390.9
(22) Date of filing: 27.05.2021
(51) Int. Cl.: A47K 5/12

(54) **SANITISER DISPENSER**

(30) Priority: 05.06.2020 AU 2020901859
(71) Applicant: Maor, Moshe, Welshpool, WA 6106 (AU)
(72) Inventor: MAOR, Moshe, Welshpool, WA 6106 / AU (AU); KHOURY, Edward Joseph, Bateman, 6150 (AU)
(74) Representative: Wittmann, Günther

(57) **Abstract**

A sanitiser dispenser (10) comprising a housing (12) including a plurality of chambers (18) each including a pouch (14) for receiving a fluid. A dispensing member (16) is secured to each of the pouches (14) such that fluid may flow outwardly from an interior of the corresponding pouch (14) through an aperture (44) in the dispensing member (16). A closure mechanism is associated with each of the pouches (14) having open and closed positions. In the open position fluid may flow through the aperture (44) and in the closed position fluid is restricted from flowing through the aperture (44). When pressure is applied to the pouch (14) with the closure mechanism in the open position, fluid is dispensed out through the aperture 44.

## Description

### Field of the Invention

The present invention relates to a device for dispensing a quantity of sanitiser and a bottle for refilling the dispenser.

### Background to the Invention

With an increase in focus on restricting spread of disease in public places, there has been a corresponding increase in the use of sanitiser to disinfect the hands or other objects. Such sanitiser is generally provided in a liquid form which can be dispensed from a conventional bottle or tube through a standard nozzle. In order to effectively sanitise the hands in public places, it is necessary to carry this type of bottle or tube around, which can be inconvenient.

The present invention relates to a dispenser having a construction such that it can be easily attached to another object in a convenient location and an associated refill bottle. The dispenser provides for easy application of a quantity of sanitiser and can be refilled and re-used as required.

### Summary of the Invention

According to one aspect of the present invention there is provided a sanitiser dispenser comprising:
a housing including a plurality of chambers each including a pouch for receiving a fluid;
a dispensing member secured to each of the pouches such that fluid may flow outwardly from an interior of the corresponding pouch through an aperture in the dispensing member; and
a closure mechanism associated with each of the pouches having open and closed positions such that in the open position fluid may flow through the aperture and in the closed position fluid is restricted from flowing through the aperture;
wherein when pressure is applied to the pouch with the closure mechanism in the open position, fluid is dispensed out through the aperture.

Preferably each of the dispensing members is connected to a neck portion of the corresponding pouch and the closure mechanism comprises rotational mounting of the dispensing member such that the dispensing member is rotatable from the closed position in which the neck portion of the pouch is folded and the open position in which the neck portion is unfolded to allow fluid within the pouch to flow into a hole in the dispensing member connected by a conduit to the aperture.

Preferably the housing comprises upper and lower walls connected by a relatively short edge wall.

In a preferred embodiment, the chambers comprise elongate chambers extending between the upper and lower walls from a first side of the housing to a second side of the housing for receiving the pouches, the pouches being elongate in shape.

Preferably the edge wall comprises a side portion connecting between the upper and lower walls adjacent the second side of the housing and first and second end portions connecting between the upper and lower walls adjacent the first and second ends respectively.

Preferably a plurality of dividing walls are provided between the upper and lower walls extending generally between the first and second sides of the housing to define the chambers.

Preferably a cross member is provided extending between the first and second end portions of the edge wall, the cross member being offset from the first side of the housing and wherein the upper wall extends from the side portion of the edge wall to the cross member to define the openings.

Preferably the neck portion of each of the pouches passes under the cross member to be adjacent the corresponding opening.

In a preferred embodiment, each of the dispensing members is connected between adjacent dividing walls, or between one of the dividing walls and the adjacent first or second end portion of the edge wall, such that the dispensing member is rotatable between the open and closed positions.

Preferably the aperture is provided in a first end of the dispensing member and the hole is provided in a first side of the dispensing member.

Preferably, in the closed position, the first side of the dispensing member is located adjacent the lower wall of the housing such that the neck portion of the pouch is in the folded configuration and, in the open position, the dispensing member extends outwardly from the first side of the housing such that the first side thereof faces upwardly away from the upper wall and neck portion of the pouch is in the unfolded configuration.

In accordance with a second aspect of the present invention, there is provided a refill bottle comprising:
first and second side walls connected around the periphery thereof by a relatively short edge wall to define a chamber for storing sanitising fluid;
a base portion in the first side wall having an applicator nozzle rotatable between a retracted position and an extended position;
a groove provided around the periphery of the first side wall to define a central wall portion;
wherein the wall portion is formed a relatively rigid material and the groove is formed from a relatively flexible material such that the central wall portion may be depressed inwardly towards the second side wall by deforming the groove in order to pump sanitising fluid out through the applicator nozzle.

Preferably the first and second side walls are circular such that the refill bottle comprises a relatively thin circular container.

Preferably the applicator nozzle includes a cylindrical base mounted for rotation within a recess in the base portion and an elongate neck extending outwardly from the cylindrical base.

Preferably the elongate neck includes a closed distal end and a hole in a side thereof from which sanitising fluid may be dispensed.

Preferably the base portion extends inwardly from the periphery of the first side wall and the groove extends around the base portion

### Brief Description of the Drawings

The invention will now be described, by way of example, with reference to the following drawings, in which:
Figure 1 is an upper perspective view of a sanitiser dispenser in accordance with the present invention;

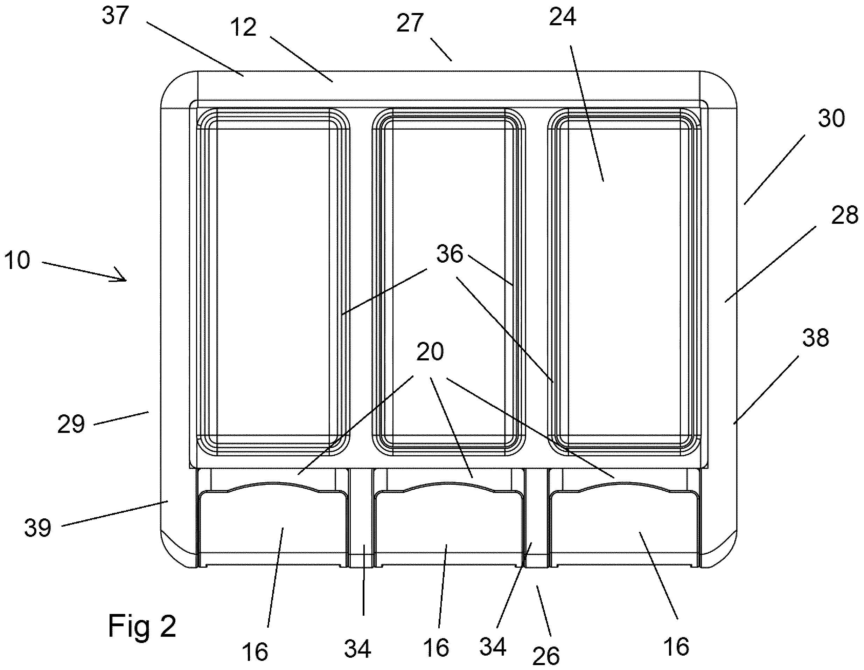

Figure 2 is a top view of the sanitiser dispenser of Figure 1;

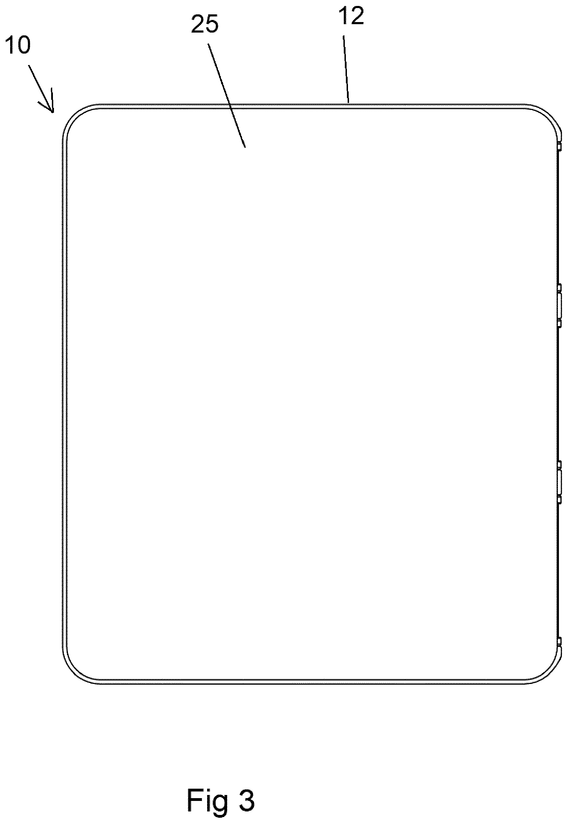

Figure 3 is a bottom view of the sanitiser dispenser of Figure 1;

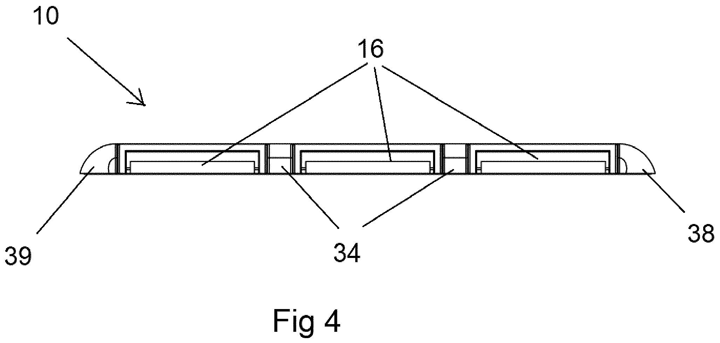

Figure 4 is a side view of the sanitiser dispenser of Figure 1;

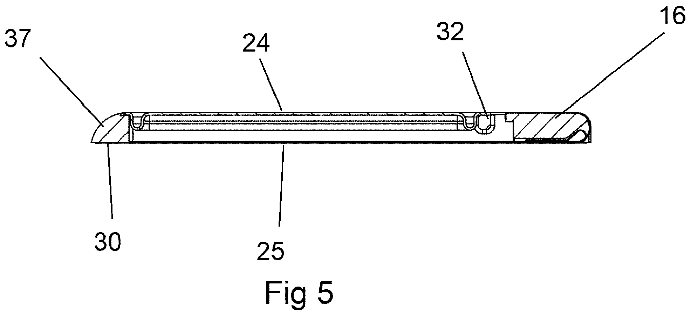

Figure 5 is an end cross-sectional view of the housing of the sanitiser dispenser of Figure 1;
Figure 6 is an exploded view of the sanitiser dispenser of Figure 1;
Figure 7 is an upper perspective view of the sanitiser dispenser with a dispensing member of one of the pouches moved to the open position;

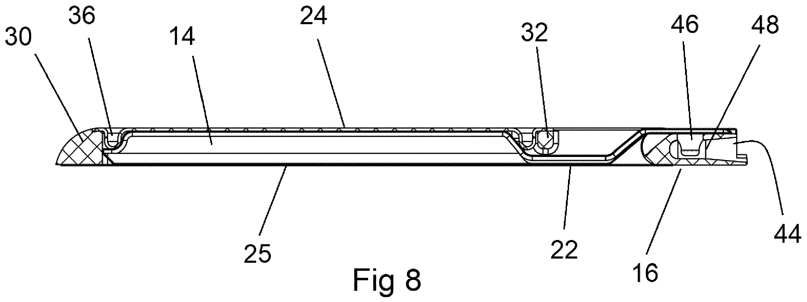

Figure 8 is an end cross-sectional view through the chamber of the pouch with the dispensing member in the open position;

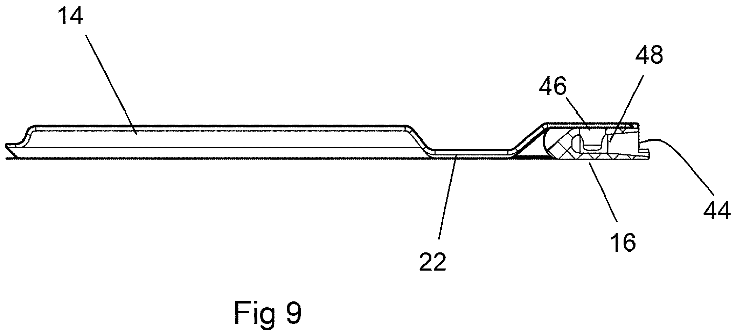

Figure 9 is a cross-sectional view of the pouch and the associated dispensing member in the open configuration;
Figure 10 is an upper perspective view showing the sanitiser dispenser secured to the rear of a mobile phone;
Figure 11 is a perspective view of a refill bottle for use with a sanitiser dispenser;
Figure 12 is a top view of the refill bottle of Figure 11;
Figure 13 an exploded view of the refill bottle;
Figure 14 is a side cross sectional view of the refill bottle;
Figure 15 is a perspective view showing the refill bottle refilling the sanitiser dispenser;
Figure 16 is a side cross sectional view showing the refill bottle refilling the sanitiser dispenser; and
Figure 17 is a close-up view of Figure 16 showing the engagement between the refill nozzle and the sanitiser dispenser.

### Detailed Description of Preferred Embodiments

Referring to the Figures, there is shown a sanitiser dispenser 10 comprising generally a housing 12 including a plurality of pouches 14. Each of the pouches 14 comprises a flexible receptacle provided for receiving a fluid, such as a sanitising fluid.

The housing 12 includes a plurality of chambers 18. Each of the chambers 18 is provided for receiving one of the pouches 14. The housing 12 includes also an opening 20 for each of the chambers 18 such that the opening 20 provides access to an end of the chamber 18. In the embodiment shown, each of the pouches 14 is elongate and each of the chambers 18 is also generally elongate such that the shape of the chamber 18 generally corresponds with the shape of the pouch 14. A neck portion 22 of each of the pouches 14, through which the interior of the pouch 14 is accessible, is located in use adjacent the corresponding opening 20 in the housing 12.

The housing 12 includes upper and lower walls 24 and 25. Each of the upper and lower walls 24 and 25 are located adjacent each other such that the housing 12 is relatively thin. In the embodiment shown, the upper and lower walls 24 and 25 are rectangular in shape such that the defined housing 12 includes first and second sides 26 and 27 and first and second ends 28 and 29.

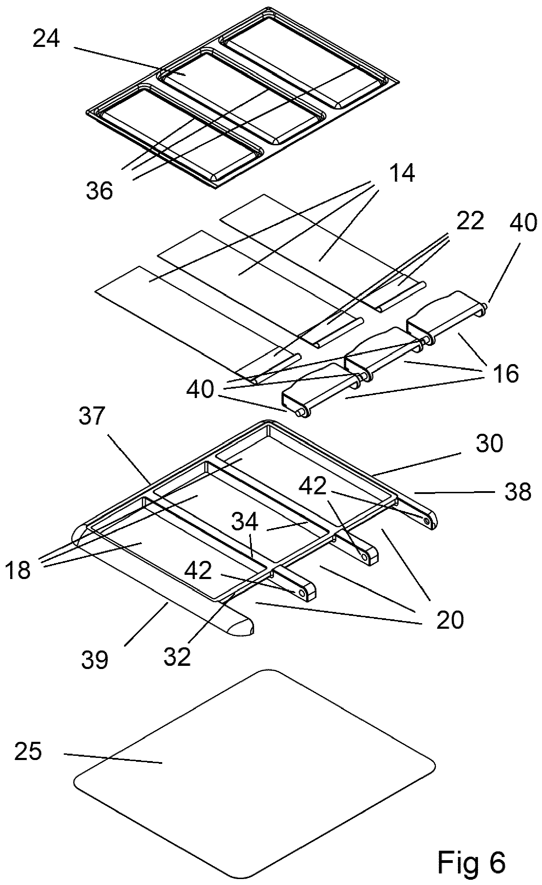

A relatively short edge wall 30 extends between the upper and lower walls 24 and 25 such that the upper and lower walls 24 and 25 are supported apart to define the interior of the housing 12. The edge wall 30 (as best seen in Figure 6) comprises a side portion 37 connecting between the upper and lower walls 24 and 25 adjacent the second side 27 of the housing 10 and first and second end portions 38 and 39 connecting between the upper and lower walls 24 and 25 adjacent the first and second ends 28 and 29 of the housing 12 respectively.

A plurality of dividing walls 34 are provided between the upper and lower walls 24 and 25 extending generally across between the first and second sides 26 and 27 of the housing 10. The dividing walls 34 define the chambers 18 such that the chambers 18 are elongate and extend between the first and second sides 26 and 27 of the housing 10. In the embodiment shown, there are provided three such dividing walls 34 to form four chambers 18 for receiving the pouches 14.

A cross member 32 is provided extending between the first and second end portions 38 and 39 of the edge wall 30. The cross member 32 is offset from the first side 26 of the housing 10 and the upper wall 24 extends from the side portion 37 of the edge wall 30 to the cross member 32 such that the upper wall 24 is narrower than the lower wall 25. The openings 20 are thereby defined by the narrower upper wall 24 and the open side of the edge wall 30 adjacent the first side 26 of the housing 10.

The cross member 32 is connected to the first and second end portions 38 and 39 of the edge wall 30 and the dividing walls 34 adjacent upper edges thereof such that a neck portion 22 of each of the pouches 14 may pass under the cross member 32 to be adjacent the corresponding opening 20.

The upper wall 24 comprises a flexible material, such as a suitable plastic material and includes a plurality of channels 36 therein. Each of the channels 18 is provided above one of the chambers 18 such that the upper wall 24 extends downwardly around the periphery of the chamber 18. The portions of the upper wall 24 within the channels 36 may thereby be more easily pressed downwardly to engage with the pouch 14 within the chamber 18.

Each of the pouches 14 is provided with a dispensing member 16. The dispensing member 16 is secured across the neck portion 22 of the pouch 14 such that the dispensing member 16 seals the neck portion 22. Each of the dispensing members 16 includes an aperture 44 in a first end thereof. Each of the dispensing members 16 includes also a hole 46 in a first side thereof. The neck portion 22 of the pouch 14 is secured to the side of the dispensing member 16 such that the interior of the pouch 14 is in communication with the hole 46. The hole 46 is connected to the aperture 44 by a conduit 48 extending through the dispensing member 16.

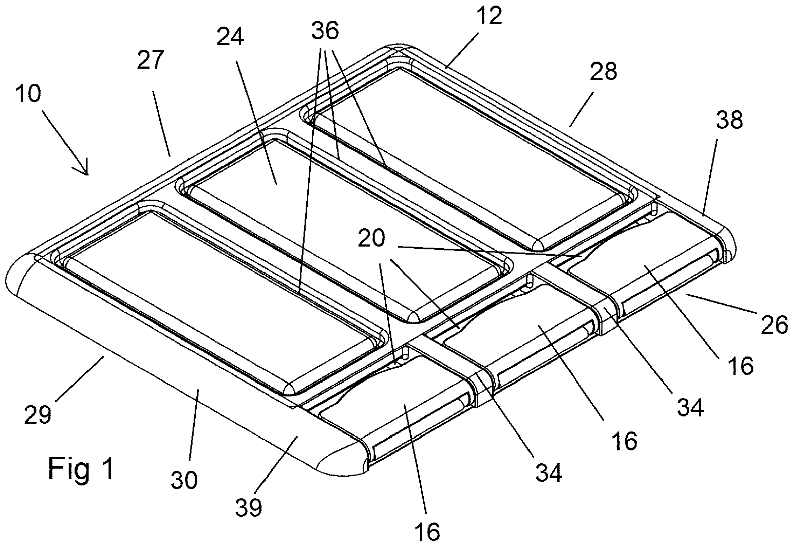

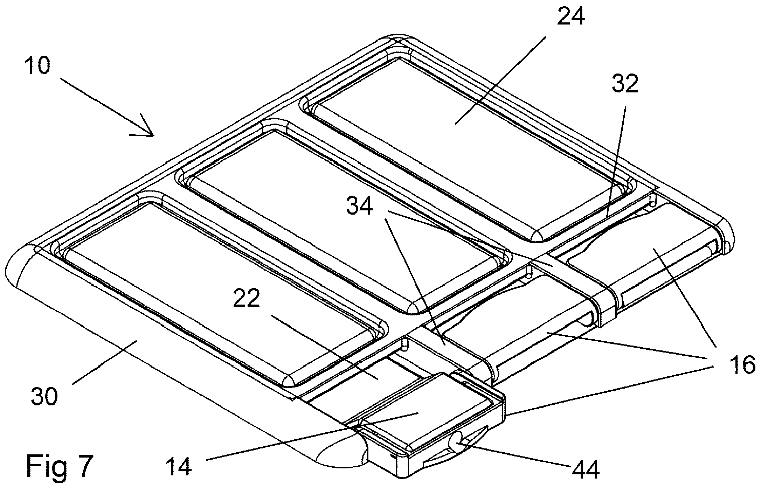

The dispensing members 16 are each provided with a closure mechanism in order to allow or restrict flow of the fluid outwardly via the dispensing member 16. Each of the dispensing members 16 is connected adjacent the opening 20 such that the dispensing members 16 is rotatable between a closed position (as can be seen for example in Figure 1) and an open position (as can be seen for example in Figure 7). Each of the dispensing members 16 is connected between the adjacent dividing walls 34, or the dividing wall 34 and the adjacent first or second end portion 38 or 39 of the edge wall 30, such that the dispensing member 16 is rotatable between the open and closed positions. In the embodiment shown, the dispensing members 16 include lugs 40 on opposite sides thereof to be received in indents 42 in the adjacent dividing wall 34 or first or second end portion 38 or 29 of the edge wall 30.

In the closed position, the first side of the dispensing member 16 is located adjacent the lower wall 25 of the housing 12 such that the neck portion 22 of the pouch 14 is in a folded configuration. In the open position, the dispensing member 16 extends outwardly from the first side 26 of the housing 12 such that the first side thereof faces upwardly away from the upper wall 24 and the neck portion 22 of the pouch 14 is in an unfolded configuration. In the closed position, the first end of the dispensing member 16 in which the aperture 44 is provided is located adjacent the cross member 32. In the open position, the first end of the dispensing member 16 is located outwardly away from the first side 26 of the housing 12.

When each of the dispensing members 16 is in the closed position, the folded neck portions 22 of the pouches 14 retain sanitiser fluid within the pouches 14. When it is required to dispense sanitising fluid from within one of the pouches 14, the dispensing member 16 is rotated to the open position to unfold the neck portion 22 of the pouch 14. The upper wall 24 of the housing 12 is formed from a slightly flexible material such that the upper wall 14 may be pressed in order to compress the pouch 14 and thereby squeeze the sanitising fluid into the hole 46 and outwardly through the aperture 44 via the conduit 48. A single dose of sanitising fluid may therefore easily be dispensed from one of the pouches 14 which then can be subsequently closed by returning the dispensing member 16 to the closed position.

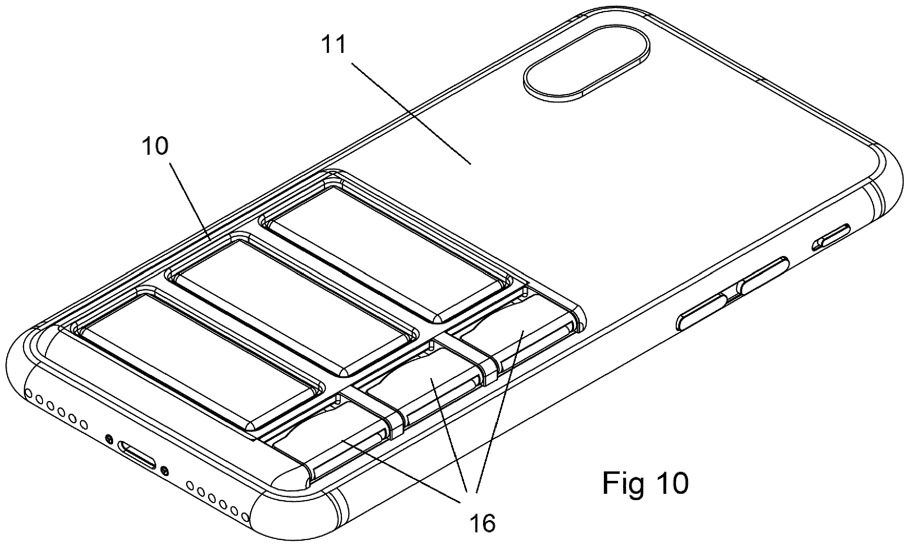

The housing 12 of the sanitiser dispenser 10 may be conveniently attached to any suitable object by securing the lower wall 25 with a suitable adhesive. The housing 12 may, for example, be secured to a rear side of a mobile phone 11 or phone case provided on the phone 11, as shown in Figure 10. The sanitiser dispenser 10 will therefore be readily available for use without the requirement for carrying a separate bottle or tube of sanitiser. The lower wall 25 may itself comprise a sheet of adhesive tape, such as double-sided tape such that the cover can be peeled off the adhesive tape layer to stick down sanitiser dispenser 10.

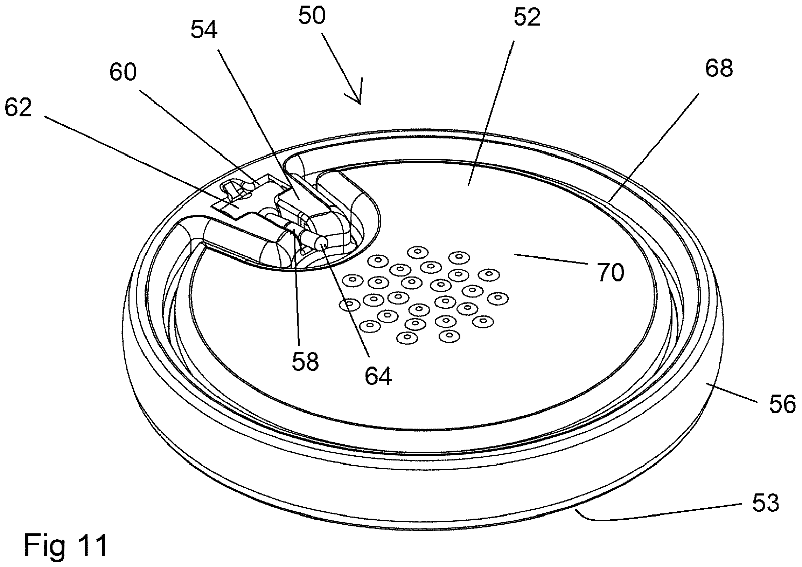

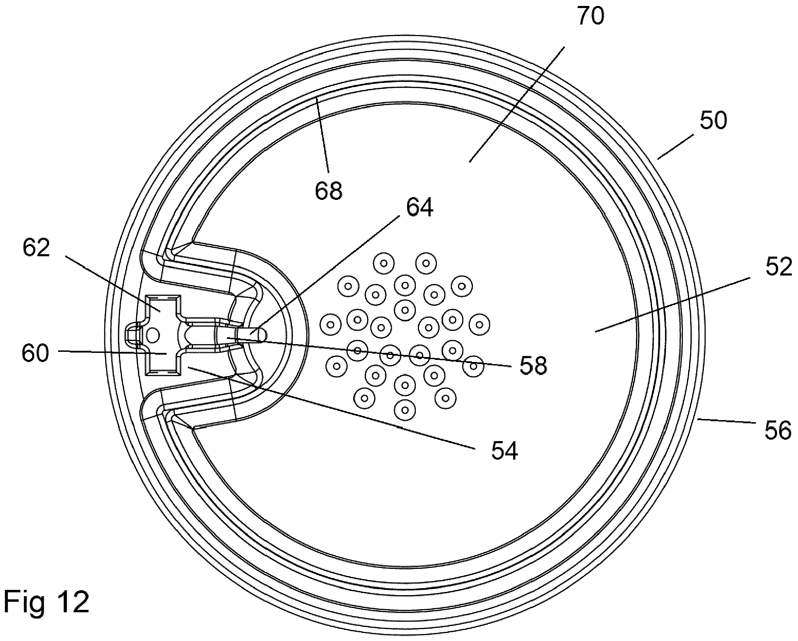

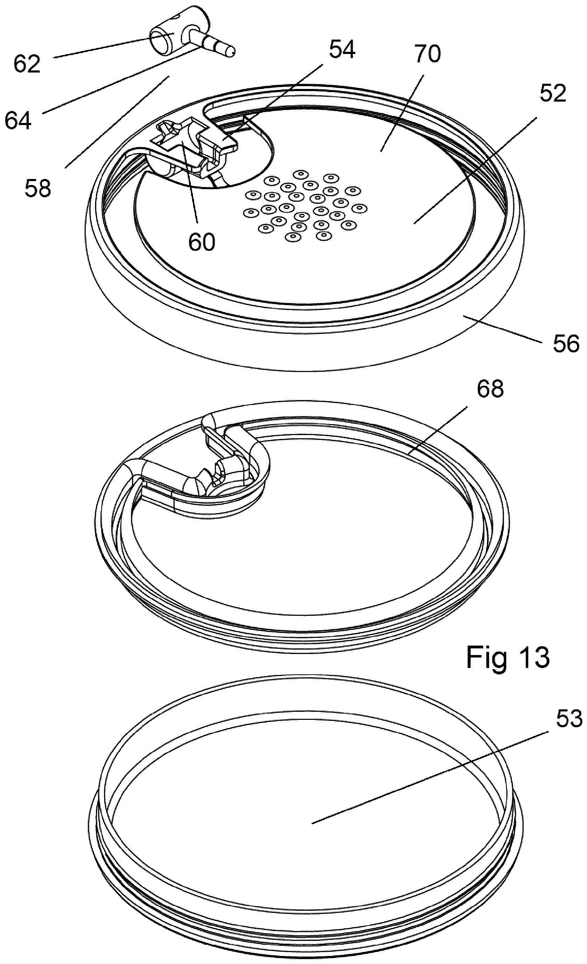

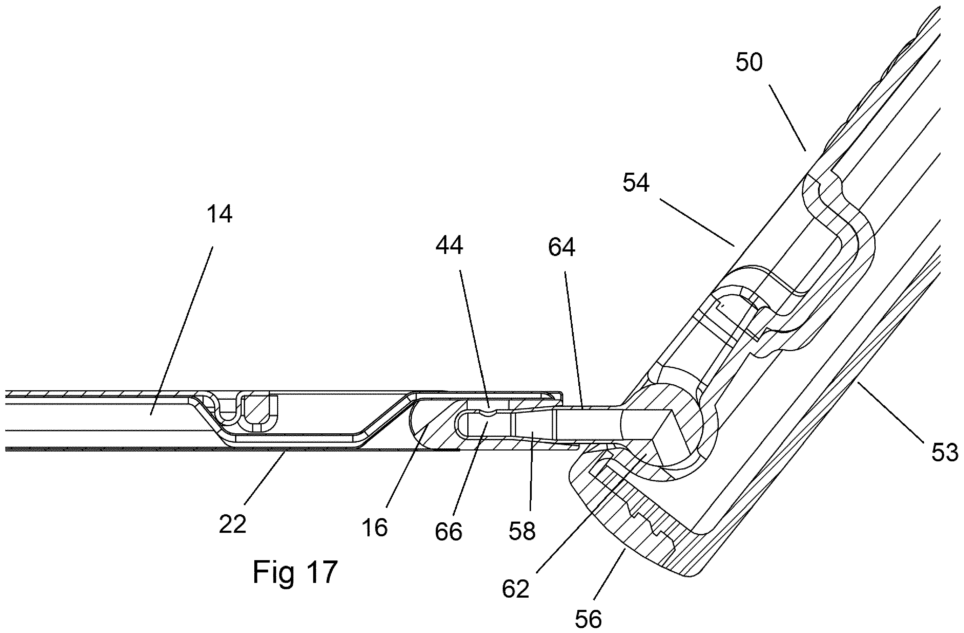

A refill bottle 50 (as shown in Figures 11 to 17) is provided for storing a larger quantity of sanitising fluid to allow refilling of each of the pouches 14. The refill bottle 50 comprises first and second side walls 52 and 53 connected around the periphery thereof by an edge wall 56. In the embodiment shown, the first and second side walls 52 and 53 are circular and the edge wall 56 is relatively short such that the refill bottle 50 comprises a relatively thin circular container.

The refill bottle 50 includes an applicator nozzle 58 from which a sanitising fluid may be dispensed in order to refill the pouches 14 of the sanitiser dispenser 10. In embodiment shown, the applicator nozzle 58 is pivotally mounted to a base portion 54 provided in the first side wall 52 of the refill bottle 50.

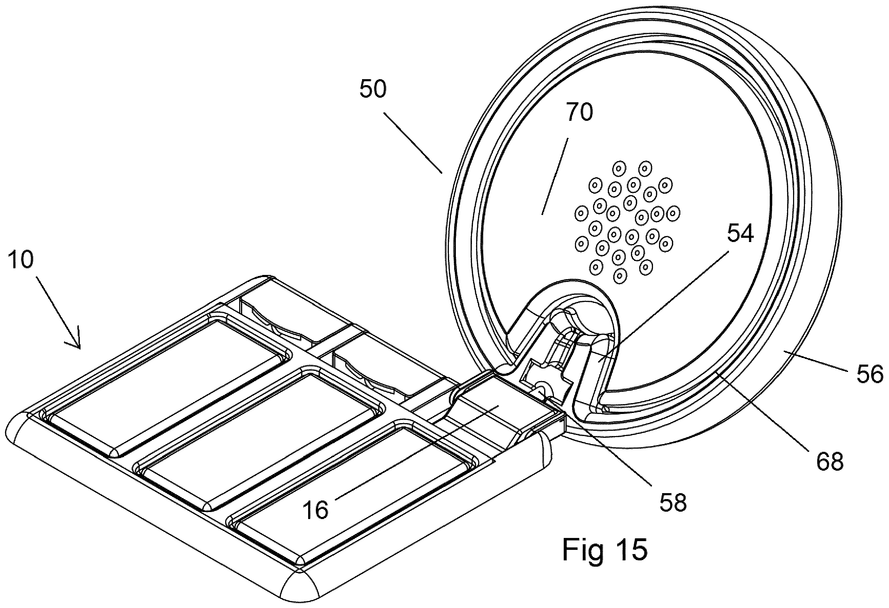

The base portion 54 extends inwardly from the periphery of the first side wall 52. An upper surface of the base portion 54 includes a recess 60 in which the applicator nozzle 58 is mounted. The applicator nozzle 58 includes a cylindrical base 62 mounted for rotation within the recess 60 and an elongate neck 64 extending outwardly from the cylindrical base 62. The applicator nozzle 58 can be rotated about the cylindrical base 62 between a retracted position (as shown in Figure 11) and an extended position (as shown in Figures 15 to 17). In the extended position, the elongate neck 64 extends outwardly from the recess 60 such that it may be received into the aperture 44 of the dispensing member 16.

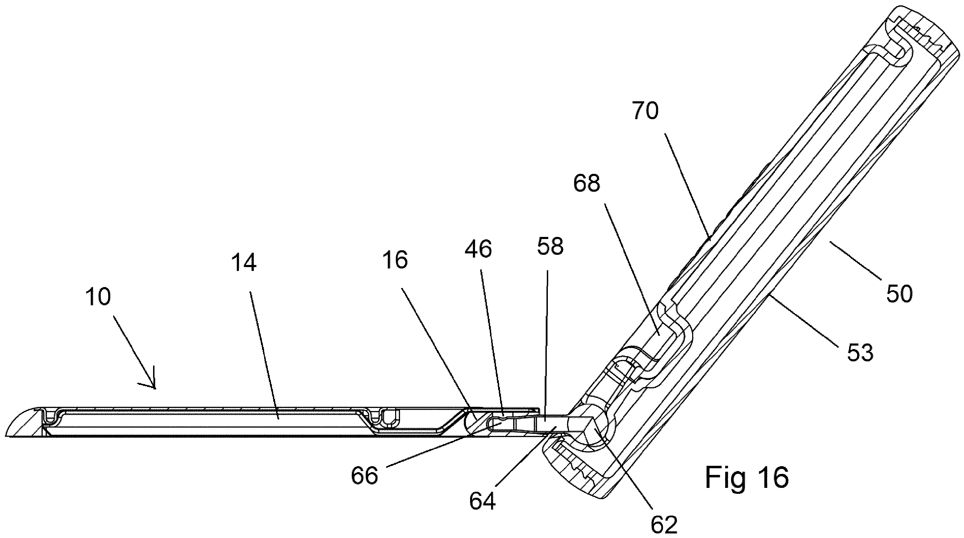

They elongate neck 64 includes a closed distal end and a dispensing hole 66 in a side thereof (as can be seen in Figure 16) such that, when inserted into the aperture 44, sanitising fluid may flow from the dispensing hole 66 in the elongate neck 64 through the conduit 48 and then out of the hole 46 into the into the pouch 14.

In a further embodiment (not shown), the pouch 14 may also include an internal tube extending from the dispensing member 16 to an end of the pouch 14 remote from the dispensing member 16. The tube is in communication with the aperture 44 such that fluid passes from the refill bottle 50 through tube to enter the interior of the pouch 14 at the end remote from the dispensing member 16. This arrangement may be provided to fill the pouch 14 from the end remote from the dispensing member 16 to reduce the likelihood of an airlock being formed while refilling of the pouch 14.

The first side wall 52 of the refill bottle 50 includes a groove 68 around the periphery thereof. The groove 68 extends around the first side wall 52 and passes around the base portion 54. The groove 68 defines a central wall portion 70 in the first side wall 52. The central wall portion 70 is formed from a relatively rigid material and the groove 68 is formed from a relatively flexible material such that the central wall portion 70 may be depressed inwardly towards the second side wall 53 by deforming the groove 68. The central wall portion 70 may therefore be depressed inwardly in order to pump sanitising fluid out through the applicator nozzle 58.

The refill bottle 50 may therefore be used to store a larger quantity of sanitising fluid and may be engaged with each of the dispensing members 16 in order to refill the pouches 14 with sanitising fluid as required.

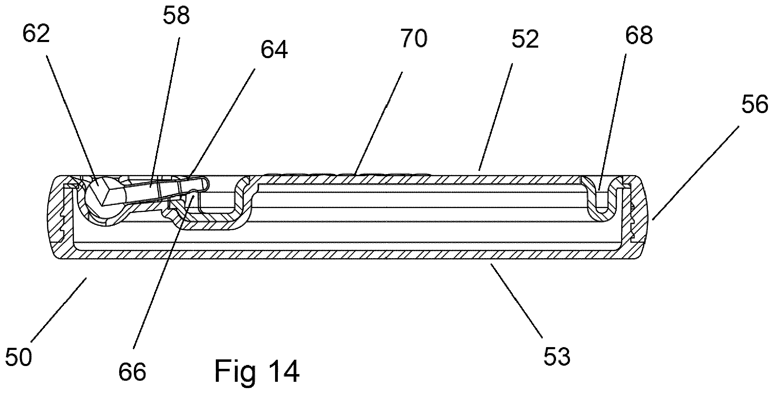

The refill bottle 50 may be provided in two parts. As can be seen in Figure 14, the first part comprises the first side wall 52 and a first portion of the edge wall 56 and the second part comprises the second side wall 53 and a second portion of the edge wall 56. The first and second parts may be connected together by threaded engagement between the first and second portions of the edge wall 56. The refill bottle 50 may then be separated into the two parts for filling. In an alternative embodiment (not shown), the refill bottle 50 may be provided with a refill opening having a removable cap.

It will be readily apparent to persons skilled in the relevant arts that various modifications and improvements may be made to the foregoing embodiments, in addition to those already described, without departing from the basic inventive concepts of the present invention.

## Claims

1. A sanitiser dispenser comprising:
a housing including a plurality of chambers each including a pouch for receiving a fluid;
a dispensing member secured to each of the pouches such that fluid may flow outwardly from an interior of the corresponding pouch through an aperture in the dispensing member; and
a closure mechanism associated with each of the pouches having open and closed positions such that in the open position fluid may flow through the aperture and in the closed position fluid is restricted from flowing through the aperture;
wherein when pressure is applied to the pouch with the closure mechanism in the open position, fluid is dispensed out through the aperture.

2. A sanitiser dispenser in accordance with claim 1, wherein each of the dispensing members is connected to a neck portion of the corresponding pouch and the closure mechanism comprises rotational mounting of the dispensing member such that the dispensing member is rotatable from the closed position in which the neck portion of the pouch is folded and the open position in which the neck portion is unfolded to allow fluid within the pouch to flow into a hole in the dispensing member connected by a conduit to the aperture.

3. A sanitiser dispenser in accordance with claim 1 or 2, wherein the housing comprises upper and lower walls connected by a relatively short edge wall.

4. A sanitiser dispenser in accordance with claim 3, wherein the chambers comprise elongate chambers extending between the upper and lower walls from a first side of the housing to a second side of the housing for receiving the pouches, the pouches being elongate in shape.

5. A sanitiser dispenser in accordance with claim 4, wherein the edge wall comprises a side portion connecting between the upper and lower walls adjacent the second side of the housing and first and second end portions connecting between the upper and lower walls adjacent the first and second ends respectively.

6. A sanitiser dispenser in accordance with claim 5, wherein a plurality of dividing walls are provided between the upper and lower walls extending generally between the first and second sides of the housing to define the chambers.

7. A sanitiser dispenser in accordance with claim 6, wherein a cross member is provided extending between the first and second end portions of the edge wall, the cross member being offset from the first side of the housing and wherein the upper wall extends from the side portion of the edge wall to the cross member to define the openings.

8. A sanitiser dispenser in accordance with claim 7, wherein the neck portion of each of the pouches passes under the cross member to be adjacent the corresponding opening.

9. A sanitiser dispenser in accordance with claim 8, wherein each of the dispensing members is connected between adjacent dividing walls, or between one of the dividing walls and the adjacent first or second end portion of the edge wall, such that the dispensing member is rotatable between the open and closed positions.

10. A sanitiser dispenser in accordance with claim 9, wherein the aperture is provided in a first end of the dispensing member and the hole is provided in a first side of the dispensing member.

11. A sanitiser dispenser in accordance with claim 10, wherein in the closed position, the first side of the dispensing member is located adjacent the lower wall of the housing such that the neck portion of the pouch is in the folded configuration and, in the open position, the dispensing member extends outwardly from the first side of the housing such that the first side thereof faces upwardly away from the upper wall and neck portion of the pouch is in the unfolded configuration.

12. A refill bottle comprising:
first and second side walls connected around the periphery thereof by a relatively short edge wall to define a chamber for storing sanitising fluid;
a base portion in the first side wall having an applicator nozzle rotatable between a retracted position and an extended position;
a groove provided around the periphery of the first side wall to define a central wall portion;
wherein the wall portion is formed a relatively rigid material and the groove is formed from a relatively flexible material such that the central wall portion may be depressed inwardly towards the second side wall by deforming the groove in order to pump sanitising fluid out through the applicator nozzle.

13. A refill bottle in accordance with claim 12, wherein the first and second side walls are circular such that the refill bottle comprises a relatively thin circular container.

14. A refill bottle in accordance with claim 12 or 13, wherein the applicator nozzle includes a cylindrical base mounted for rotation within a recess in the base portion and an elongate neck extending outwardly from the cylindrical base, the elongate neck including a closed distal end and a hole in a side thereof from which sanitising fluid may be dispensed.

15. A refill bottle in accordance with any one of claims 12 to 15, wherein the base portion extends inwardly from the periphery of the first side wall and the groove extends around the base portion.
